# EUROPEAN PATENT APPLICATION

(11) **EP 4 800 700 A1**
(43) Date of publication of application: **02.09.2026**
(21) Application number: 25160531.7
(22) Date of filing: 27.02.2025
(51) Int. Cl.: G16H 30/40, A61B 6/00, G16H 40/63, G16H 50/70

(54) **PERSONALIZED IMAGE QUALITY AND X-RAY DOSE OPTIMIZATION USING MACHINE LEARNING**

(71) Applicant: Koninklijke Philips N.V., 5656 AE Eindhoven (NL)
(72) Inventor: LELY, Arnoud Hendrik, Eindhoven (NL); ENGEL, Klaus Jürgen, Eindhoven (NL); PETERS, Inge Mareike, Eindhoven (NL)
(74) Representative: Philips Intellectual Property & Standards

(57) **Abstract**

System (FS) and related method of facilitating operation of an imaging apparatus (IA). The system comprises an input interface (IN) for receiving input data (D) comprising i) imaging context data (c) relating to an intended imaging procedure or to a phase of such a procedure and/or to a subject (PAT) to be imaged, and ii) image quality, IQ, score data (s) indicative of an intended IQ to be achieved. A trained machine learning model (M) of the system processes the input data (D) to compute output data (*p*) related to an imaging setting for the imaging apparatus (IA).

## Description

### FIELD OF THE INVENTION

The invention relates to a system for facilitating imaging operation of an imaging apparatus, to an imaging arrangement including such a system, to a related method, to a training system for training a machine learning model for use in such a system, to a related machine learning model training method, to a training data provider system capable of providing data for the training system, to a related method of providing such training data, to a computer program element, to a computer readable medium, and to a use of such machine learning model.

### BACKGROUND OF THE INVENTION

Medical imaging is a first-line mainstay of medicine, at least since discovery of X-rays by Wilhelm Roentgen in the 19th century. Being able to see "inside" a patient in a non-invasive manner is invaluable for diagnosis, therapy, planning and other medical tasks.

Medical imaging equipment, such as a C-arm imager, a CT (computed tomography) scanner, or MRI (magnetic resonance imaging) is however complex machinery. Its correct operation to achieve the imaging quality ("IQ") needed of the task at hand is no mean feat. There may be many different settings, parameters, adjustments, etc., that command mastery. Even experienced operators may get it wrong, at times, be it due to time pressure, fatigue, etc., let only novice users, such as those in residence, early years fellows, etc.

### SUMMARY OF THE INVENTION

There may therefore be a need for improved, efficient imaging or handling of imaging equipment, in particular in the medical field.

An object of the present invention is achieved by the subject matter of the independent claims where further embodiments are incorporated in the dependent claims. It should be noted that the following described aspect of the invention equally applies to the imagining arrangement including such a system for facilitating imaging operation, to the related method, to the training system for training the machine learning model for use in such system, to the related machine learning training method, to the training data provider system capable of providing data for the training system, to the related method of providing such training data, to the computer program element and to the computer readable medium.

In one aspect there is provided a system of facilitating operation of an imaging apparatus, comprising:-
input interface for receiving input data comprising i) imaging context data relating to an intended imaging procedure or to a phase of such a procedure and/or to a subject to be imaged, and ii) image quality, IQ, score data indicative of an intended IQ to be achieved, and
a trained machine learning model configured to process the input data to compute output data related to an imaging setting for the imaging apparatus.

The score may be interpreted as a qualitative descriptor. It may relate to an image quality (IQ) "flavor", that is, a certain IQ preference, of an entity (individual user, or collective entity, such as an imaging department, a hospital, clinic, etc.) using the imaging apparatus. Whilst there may be one or more optimal such imaging setting given i),ii), in some use cases, as may be assumed herein, such imaging setting, in particular parameters to which such setting is based, may be conceptualized as a system of one or more numerical ranges in an "imaging parameter space". Some or each such imaging setting that falls withing the system of ranges may be considered clinically acceptable/optimal.

In embodiments, the said score represents an imaging preference from a scale of such preferences of a user, department, or organization. The organization may be a clinic, hospital, GP practice, imaging/radiology department, etc.

In embodiments, the input data is so receivable in an ongoing imaging procedure, and wherein the imaging setting is one for acquisition of a next frame in the ongoing imaging procedure.

In embodiments, the imaging context as per the input data is subject to change in the ongoing imaging procedure, wherein the trained machine learning model to compute updated such imaging settings per such change.

Thus, the system may be used in dynamic /real time imaging setups, such as in interventional procedures. Thus, imaging settings in interventional imaging are in general to subject to repeated, in some cases quasi-continuous, adaption to better capture the region of interest ("ROI"), and/or intervention(s) performed in relation to such ROI.

In embodiments, the said input data is suppliable by user via a user interface.
The user interface may be any one of a graphical user interface (GUI), and/or an interface that captures and responds to speech, gesture, etc.

In embodiments, the system includes an output interface for supplying the said imaging setting.

Whilst imaging setting may be supplied by the system automatically on input, in embodiments the system may allow user to overrule (via the, or a, user interface) the supplied imaging setting.

The supplied imaging setting may be applied automatically or may be provided to user first (e.g., via displaying, sounding out, or in whichever way), and it is the user that then causes the imaging setting to be applied. User may reject the proposed setting, and instead adjust the setting manually. The output interface may supply an indication of the imaging setting for the intended IQ for the next frame (run).

In embodiments, the output interface is capable of interfacing with control circuitry of the imaging apparatus to thereby cause the imaging setting being applied to the imaging apparatus, thus enabling the imaging apparatus to acquire one or more frames at the said imaging setting.

In embodiments, on the system so supplying the imaging setting and the imaging apparatus acquiring a current frame at the supplied imaging setting, the input interface then to receive further input data including a user feedback on the current frame, and the trained machine learning model further capable to compute, based on the feedback, a new imaging setting for a subsequent frame.

In embodiments, the imaging apparatus is of the interventional type.

In embodiments, the imaging apparatus is X-ray based.

In embodiments, the procedure is an image-guided procedure.

In another aspect there is provided an imaging arrangement, comprising at least parts of the system as per any one of the preceding claims, and further comprising any one or more of: the imaging apparatus, the user interface, data storage on which is stored at least some parameters of the trained model.

In another aspect there is provided a computer-implemented method of facilitating operation of an imaging apparatus, comprising:-
receiving input data comprising i) imaging context data relating to an intended imaging procedure or to a phase of such a procedure and/or to a subject to be imaged, and ii) image quality, IQ, score data indicative of an intended IQ to be achieved, and
by trained machine learning model, processing the input data to compute output data related to an imaging setting for the imaging apparatus.

In another aspect there is provided a method of training, based on training data, a machine learning model to obtain the model as per in the system of models of any one of the above mentioned aspects or embodiments.

In another aspect there is provided a method of providing training data on which the model is trainable. This method may include causing displaying of training imagery, and receiving scoring data from user on viewing the displayed imagery. The respective imaging setting that was in effect when the training imagery was acquired and the corresponding context under which the imagery was so acquired may be assumed known/controllable. The providing of the training data may include providing the context data and score in association with the respective imaging setting.

In another aspect there is provided a computer program element, which, when being executed by at least one computing system, is adapted to cause the computing system to perform the method as per any one of the above mentioned aspects.

In another aspect there is provided at least one computer readable medium having stored thereon the saif program element.

The proposed system addresses a general problem of optimization of image quality and X-ray dose, which is the fact that only the human (clinical) user can determine what level and said "flavor" of image quality is needed to optimize the clinical task (or task in other domains than medical) at hand, because humans have different (and very individual) perceptions of what is "a good image quality". Furthermore, certain events or tasks during an intervention suggest switchable dose levels to reduce (e.g., optimize) image quality, and thus reduce X-ray exposure. Dose levels can easily vary by a factor of 4 between different dose settings in some systems and often users request for even larger differences for the same kind of clinical procedures depending upon complexity of the intervention. The proposed setup which accounts for user image quality preference (the said score), is superior to some current system where many settings, such as dose level, are selected manually by user, such as by selecting one of the available pre-defined image quality and/or dose levels which may not reflect or comport with the specific user preferences.

The proposed system helps avoiding unnecessary retakes. Whilst the user may know their IQ preferences, they may not necessarily know what imaging settings to adjust, especially when dealing with unfamiliar equipment, but not only. But even if they know the imaging equipment, they may not always know the exact imaging setting for each view, anatomy, situation etc., especially when used in a dynamic IGP ("image guided procedure") setting. There is a risk the user keeps readjusting/ retaking, etc., on their "quest" to put into practice their preferred imaging setting. This is disadvantageous: not only may this harm patient and staff, e.g. in X-ray where dose is incurred with each exposure. It may also put avoidable strain on imaging equipment. For example, in X-ray imaging, the X-ray anode disk or the cathode in an X-ray tube is subject to considerable temperature gradients. Unnecessary retakes may result in premature wear out of anode disk or cathode, and thus in disruptions because of maintenance downtime, etc. The proposed setup allows avoiding this, or at least allows reducing risk for such scenarios to materialize. This is because the proposed system helps user to more quickly find the setting that corresponds to their IQ preference, and thus allows user to get the imagery at the IQ they seek right first time. It is at least in this sense that the system helps safeguarding patient/staff health, and prolong service life of possibly expensive imaging equipment, a consideration in particular in cash-strapped national health systems.

The system may be used in particular in imaging setups where there is demand for rapid and/or frequent changeovers of imaging settings, such is in said IGPs. Such imaging settings may include in particular imaging geometry changeovers. Updated imaging settings may be provided by the system in an ongoing IGP, subject to user defined personalized IQ scoring for user guided output. The system is more likely to output settings that result in imagery in line with user's personal IQ preference. User may supply this score along with imaging context data via a suitable user interface, such as a GUI, possibly touchscreen supported, or in any other way, such as via pointer tool input, keyboard input, or via a request button or similar in a control UI, such as joystick, foot pedal, etc. Such a provided personalized IQ score may be maintained throughout the IGP. However, it also envisaged in some embodiments (but not all) to dynamically update score at certain phases in the IGP, at user request. For example, in navigation phase imagery as per a first score may be preferred, but in another phase, such as deployment phase of a tool, device of implement under image guidance, imagery at another score may be preferred, etc. However, such an update is not always needed. The 'system is aware of the context, including procedure stage and should automatically adapt the imaging settings to the 'objective' IQ needed to reach a "subjective IQ score' which is still in the feasibility range (optimized IQ, in the sense that it returns acceptable imaging at lower (e.g., lowest) X-ray dose). In exceptional cases or when explicitly 'personalizing' the system, the mentioned user interaction for updating score may be an option. However, for better usability in clinical, or ex -medical applications, the system automatically selects the imaging settings. Thus, the score can be obtained from user profile data or otherwise, but may remain constant during the procedure. The context data may be updated during the imaging, thus yielding different imaging setting. The desired IQ is generally in the feasibility range ('optimal IQ'). In embodiments, the system adapts for this "optimal IQ", as itis the subjective IQ score which the system is striving for. The subjective IQ is in general constant, namely 'just good enough for the purpose of the imaging'. The objective IQ (referred to as "q" below) on the other hand may differ substantially.

The proposed system may be used in X-ray system for interventional guided therapy systems for example. The trained machine learning model is operative to propose a imaging setting, and thus implicitly an image quality, for the next X-ray exposure based upon context data such as any one or more of: i) type of procedure, patient size, stage of the procedure and e.g. devices being used, contrast media settings, patient physiology, image quality of previous exposures, and ii) personal preference (the score) of the user.

In embodiments, the system supports a human-ML-interface, allowing the user to communicate with the model on imaging settings relevant for exposure parameters (being potentially different to default assumptions), e.g., increased image sharpness, increased contrast of devices, or the said preferred personal IQ score for better image interpretation. The said personal IQ score may be thought of as a certain IQ "flavor", that is, a kind of IQ preference.

The proposed system and method may be understood as a concept of using certain input parameters and an "indirect training method" for the ML, together with a learned relationship between: -
- clinical circumstances and, optionally, a required objective IQ;
- subjective IQ (which may depend on clinical task);
- imaging parameters (depending on patient, projection, etc.)'.

### Definitions

In general, the term *"user"* is employed herein instead of "user entity" as a shorthand. Thus, "user" may refer to a single individual clinical user, or to a group of such individual users, such as members of an imaging department, a clinic, hospital, or other medical facility.

The term *"score"* as developed herein relate to imaging settings/IQ preferences of a given user. If the user is a group, the score may be arrived at for the group by consensus, voting, standard setting committee for the groups, or similar. "User" in general refers herein to the operator of the imaging equipment, or, if an autonomous setting is used, the user may oversee, or is otherwise responsible, for the imaging. The user may be a medical professional, an interventional radiologist, a technician, etc. In general, the user is not the subject (e.g. a patient) being imaged or who is to be imaged.

*"Image quality"* (IQ) as used herein may appertain to image features of imagery obtainable herein upon acquisition of such imagery using an imaging apparatus operable at imaging settings (imaging parameters) as described herein. The said image features may relate to the image's appearance if displayed, relative to a reference view setting. In addition, or instead, it may relate to measurable image quality metrics, such as signal-to-noise ratio, contrast, sharpness, etc. Such metrics can be described by spatial statistics, or whichever way. IQ of a certain given image may also be described in terms of imaging settings used to operate imaging apparatus when acquiring the said given image. Whilst each imaging setting may yield imagery of a certain IQ, for a given IQ there may be plural imaging settings that give rise to approximately the same IQ. In general, IQ will relate to features that derive from the manner of spatial distribution of image values (pixels or voxels). The image quality may relate to such quality features for projection imagery in projection domain, or to such features of tomographic or otherwise reconstructed imagery reconstructed from projection imagery into 3D image domain, or otherwise derived imagery such as spectral imagery. The term image quality as used herein may be distinguished herein as "objective image quality" and "subjective personal (preferred) image quality", the latter being measurable and parameterized by the proposed scoring, whilst the former can be measured by any one or more of said metrics. Thus, objective image quality is represented by the said image structure metrics and their values, whereas the latter, personalized or subjective image quality, of main interest herein, appertains to a certain preference of a user entity to imagery having objective quality features that are not represented by a single imaging setting, but a set of such imaging settings. This set may be conceptualized as a cluster of points located in a certain portion of an imaging setting space. Such imaging settings space may be understood as a, possibly high dimensional, vector space made up of points with co-ordinates pertaining to the imaging settings. A certain user entity, such as an individual (human) medical user, department, group of users, hospital, or any other, may prefer a certain personalized IQ and hence an associated set of imaging settings over another set in said imaging settings space. Those imaging settings may form clusters of points in that space. The user entity may have such preference due to, for example, experience in having handled cases successfully, based on imagery having such IQ. *"Imaging setting(s)"* may be used herein in singular or plural. In either case, the term may relate to a setting for a single component of the imaging apparatus or to plural such components, depending on data encoding, representation, etc.

In addition, or instead of the above, the term *"image quality",* in particular in relation to the objective image quality, may appertain also to qualities beyond such image structure metrics and may include other considerations or aspects such as the adequacy of the image for a certain purpose for which the image is intended. Thus, the image quality may not only be a function of such image structure metrics but may in addition be a function of the intended medical or other purpose of the imagery. In addition, or instead, the image quality as used herein, either objective or subjective, may further appertain to one or more expenditures (in whichever form) that need to be expended in order to acquire the image. Dose may be one such expenditure of consideration. Thus, image quality may also appertain to the dosage incurred by a patient and/or attending staff, in particular, in interventional imaging settings as mainly envisaged herein, as does the nature of any contrast boosting measure that may be needed. Thus, such expenditure may include, in X-ray, the radiation dosage, but also amount (volume or weight, concentration, etc.) of contrast agent administered to the patient in order to boost contrast.

Thus, image quality as may be used herein may consider the structural features of the image in relation to the expenditures in terms of dose, contrast agent or similar measures that have possibly negative effects on staff and/or patient. In short, image quality as used herein is not confined to structural features of the image but also relates to acquisition expenditure of the imagery, balanced against structural features of the imagery. Thus, an image, if having desirable structural features, may still have a low IQ, if the expended contrast agent or dose is considered too high. Thus, IQ, when including such consideration of expenditure, may be referred to herein as relative or balanced IQ. However, such consideration of expenditures etc., are optional herein.

The said IQ score may be a personal score of a specific user entity. The said IQ score may be thought of as relating to an "optimal/optimized", or at least improved IQ, in the sense that the subjective/personal IQ score may still follow certain general medical imaging guidelines, principles, or paradigm, etc., such as ALARA ("good enough for the clinical task" - thus, good enough IQ, but not at too high of X-ray dose).

The *"subject"* of the imaging (the entity that is imaged) will be referred to herein mostly as the *"patient",* and may refer to herein to the whole of the said subject/patient or only a part thereof (region of interest). Having said that, "subject" may instead refer to an animal (or part) such as in a veterinary context, or indeed to inanimate objects, such as in material testing, examination of machinery, caves, plumbing, archaeological artifacts/sites, etc.

In general, the term *"machine learning"* includes a computerized arrangement (or module) that implements a machine learning ("ML") algorithm. Some such ML algorithms operate to adjust a machine learning model, thus configuring same to perform ("learn") a task. Other ML operate direct on training data, not necessarily using such an explicit model, thus in such cases the training data may form the model, or may be part of such model. This adjusting or updating of the model is called "training". In general, task performance by the ML model may improve measurably with training experience. Training experience may include suitable training data, and exposure of the model to such data. Task performance may improve, the better the data represents the task to be learned. Training experience helps improve performance if the training data well represents a distribution of examples over which the final system performance is measured. See for example, T. M. Mitchell, "Machine Learning", page 2, section 1.1, page 6 1.2.1, McGraw-Hill, 1997. The performance may be measured by objective tests based on output produced by the model in response to feeding the model with test data. The performance may be defined in terms of a certain error rate to be achieved for the given test data.

### BRIEF DESCRIPTION OF THE DRAWINGS

Exemplary embodiments of the invention will now be described with reference to the following drawings, which, unless stated otherwise, are not to scale, wherein:
Fig. 1 shows a schematic block diagram of a medical imaging arrangement;
Fig. 2 shows a component pipeline for an imaging operation of an imaging apparatus in applicable imaging settings;
Fig. 3 shows a schematic illustration of imaging parameters in a parameter space, clustered, parameterized according to personalized image scores and their use in machine learning;
Fig. 4 shows a block diagram of a facilitator system as may be used to operate an imaging apparatus;
Fig. 5 shows a training system for training a machine learning model based on training data;
Fig. 6 shows a flow chart of a computer-implemented method of facilitating operation or of an imaging apparatus; and
Fig. 7 shows a flow chart of a computer-implemented method of training a machine learning model based on training data.

### DETAILED DESCRIPTION OF EMBODIMENTS

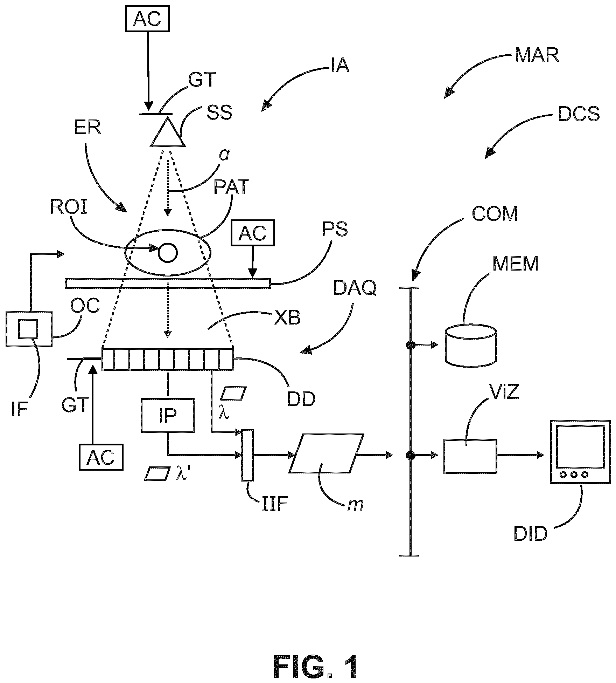

Reference is now made to the block diagram of Fig. 1 which shows a medical imaging arrangement MAR. Broadly, the arrangement MAR includes a preferably medical imaging apparatus IA. The imaging apparatus IA (for brevity referred to herein as "imager") is operative, in one or more imaging sessions, to obtain medical imagery *m.* In preferred embodiments, the medical imagery *m* so obtained may be displayed in essentially real-time on on-site, preferably on acquisition, to the clinical user of the imaging apparatus IA, such as in interventional or similar setups (to be described in more detailed below) that rely on live imagery. The imagery m may be passed through wired, wireless, or hybrid, telecommunication network COM, or in any other way to a data consumer DCS. More "hands-on" data transfers options such as via removable storage, dongles, etc., are not excluded herein.

The data consumer section DCS may include memory MEM on which the imagery M is stored, such as for later reference, radiological review ("reading"), etc. A visualizer VIZ may be operative to produce visualizations of imagery which may be displayed on a display device DID. The imagery may inform therapy, planning and/or diagnosis in a medical setting. The imagery may provide "guidance" such in image guided procedures, as will be explained in more detail below. The imagery may be reviewed by a reviewer such as a radiologist or other medical professional. Other data consumers in section DSC may include an image post-processing module, such as for segmentation, annotation, etc. (not shown). Whilst in the present main reference is made to the medical domain, this is not necessarily at the exclusion of applications in other domains.

The medical imagery *m* obtained by the imager IA is preferably representative of internal structures, organs, etc., of the patient PAT who is to be imaged in an image session by imaging apparatus IA. Thus, the medical imagery *m* allows for a non-invasive manner in which to "look" inside the patient, and to so inform diagnostic, therapeutic, planning strategies among other application scenarios.

Broadly, and as will be explained in more detail below, what is proposed herein is an imaging facilitator IF, a computing arrangement, that allows a user to easily obtain imaging settings for use with the current patient PAT and imager IA, and an intended imaging task at hand. Thanks to the imaging facilitator IF, even a novice user may be able to operate the imager IA safely and efficiently to obtain imagery *m* of sufficient image quality.

With more detailed reference to the imaging apparatus IA, this includes a data acquisition unit DAQ that is operable to acquire measurements λ during the imaging session with suitable detector device DD equipment (in the following also referred to simply as "detector DD"). The measurements λ may include variations of a measurement signal to which the patient is exposed to. The measurement signal is thought to correlate with spatial or functional features of structures of interest (also referred to as region of interest (ROI) ) within the patient. The measurement may include intensity measurements λ, e.g., projection raw data, such as may be utilized in computed tomography or radiography, as needed. In X-ray, the detector DD may be configured for digital-at-source acquisition, such as flat panel type detector, or may be of any other suitable type.

Before proving more in-depth detail on the imaging facilitator IF, more detailed reference is first made to the imaging apparatus, its components and the imaging context more generally, in order to so better assist the latter, more detailed explanations on the functioning of the said imaging facilitator IF.

Referring now in more detail to imager IA, this may include a signal source SS and the detection device DD.

The signal source SS generates a signal, for example an interrogating signal XB, which interacts with the patient to produce a response signal which is then measured by the detector device DD and converted into the measurement data λ such as the said medical imagery.

One example of the imager IA is an X-ray based imaging apparatus such as an interventional imager, radiography apparatus, configured to produce protection imagery. Volumetric tomographic (cross sectional) imaging is not excluded herein, such as via a C-arm imager or a CT (computed tomography) scanner, or via other tomographic modality.

During an imaging session, patient PAT may reside on a patient support PS (such as patient couch, bed, etc.), but this is not necessary as the patient may also stand, squat or sit etc. in an examination region ER during the imaging session. It is sufficient for the region of interest to so reside in the examination region ER/field-of-view FOV of imager IA. The examination region is formed by the portion of 3D space between the signal source SS and the detector device DD.

For example, in a C-arm interventional imaging setting, or a CT setting, acquisition is multi-directional. For example, during imaging session, X-ray source SS rotates around the examination region with the patient in it to acquire projection imagery λ from different directions α. The projection imagery is detected by the detector device DD, in this case an X-ray sensitive detector. The detector device DD may rotate or angulate around the examination region, together with the X-ray source SS. In relation to CT such co-rotation is not necessarily required, such as in CT scanners of 4^{th} or higher generation. The signal source SS, such as an X-ray source (X-ray tube), is activated so that interrogating signal in form of an X-ray beam XB issues forth from a focal spot in the tube during or in between rotation(s).

The beam XB traverses the examination region and the patient tissue therein. and interacts with same to so cause modified radiation to be generated. The modified radiation is detected by the detector device DD as intensities. The detector DD device is coupled to acquisition circuitry such as DAQ to capture the projection imagery, preferably digitally.

The same principles apply in (planar) radiography, only that there is no rotation of source SS during imaging. In such a radiographic setting, it is this projection imagery that may then be examined by the radiologist. In the tomographic/rotational setting, the muti-directional projection imagery is processed first by an image processor IP, that may run a reconstruction algorithm, that transforms projection imagery from projection domain into sectional imagery in 3D image domain. Image domain is located in the examination region ER. In other setups, though not necessarily tomographic, there may still be some processing at processor IP, such as an in spectral imaging to obtain projection imagery λ' with material-specific contrast. In other cases, the image processor IP may apply a phase-retrieval algorithm, such as in phase contrast or dark field imaging to obtain phase contrast or dark field imagery λ'. Projection imagery or reconstructed imagery will not be distinguished herein anymore, but will simply be referred to collectively as obtained imagery m made available via imaging output interface IFF, which may or may not include image processing by image processor IP. However, what is envisaged herein in the main is interventional projection imaging and source imagery m will thus mainly refer to projection imagery.

The so obtainable imagery m may however not necessarily result from X-ray imaging, although this is mainly envisaged herein. Other imaging modalities, such as emission imaging, as opposed to the previously mentioned transmission imaging modalities, are also envisaged herein such as SPECT or PET, etc. In addition, magnetic resonance imaging (MRI) is also envisaged herein in some embodiments.

For example, in MRI embodiments, the signal source SS is formed by radio frequency coils which may also function as detector device(s) DD, configured to receive, in receive mode, radio frequency response signals emitted by the patient residing in a magnetic field. Such response signals are generated in response to previous RF signals transmitted by the coils in transmit mode. There may be dedicated transmit and receive coils however in some embodiments instead of the same coils being used in the said different modes.

In emission/nuclear imaging, the source SS is within the patient in the form of a previously administered radio tracer which emits radioactive radiation that interacts with patient tissue. This interaction results in gamma signals that are detected by detection device DD, in this case gamma cameras, arranged preferably in an annulus around the examination region where the patient resides during imaging.

Instead of, or in addition to the above-mentioned modalities, ultrasound (US) is also envisaged, with signal source and detector device DD being suitable acoustic US transducers.

The imagery m generated by whichever modality IA may be passed through a communication interface COM to a (non-volatile) memory MEM, where it may be stored for later review or other processing. The imagery may be rendered into a visualization by visualizer VIZ on a screen of a display device DID. However, an online setting is not excluded herein, where the imagery is consumed in whichever way as it is produced by the imaging apparatus IA.

Imagers IA of different modalities are envisaged herein, such as X-ray based (radiography, CT (computed tomography), C-arm, conebeam/dental CT, etc.), MRI magnetic resonance imaging), nuclear imagers such as SPECT (single photon emission tomography)/PET (positron emission tomography), optical coherence tomography (OCT), ultrasound (US), etc.

In particular imagers of the tomography type (CT, MRI, OCT, nuclear), there is spatial domain between the signal source SS and the detection device DD that includes an examination region ER in which the patient, or at least part of the patient (the ROI) to be imaged, resides during imaging/data acquisition.

As said, and in more detail, the examination region ER is a portion of 3D space. Conceptually, the examination region ER can be thought of as made up of a spatial grid of spatial points (voxels). Such voxels help define tomographic sectional imagery. As will be explained in more detail below, whilst in some occasions the measurement data are of interest in their own right such as radiography, in some cases the measurements are further processed computationally to populate the grid of spatial points, voxels, with image values, to so build up a volumetric sectional image in the examination region. In this connection, the examination region is also sometimes referred to as the imaging domain, in distinction to the projection domain that is defined by a radiation sensitive surface made up of detector pixels of the detection device DD. Projection imagery (a visualization of the measurements λ as such) on the other hand, of interest in radiography such as in "chest X-ray", or other, are spatially located in the said projection domain, and not in image domain. Having said that, tomographic imaging is optional herein, as projection imagery is mainly envisaged herein.

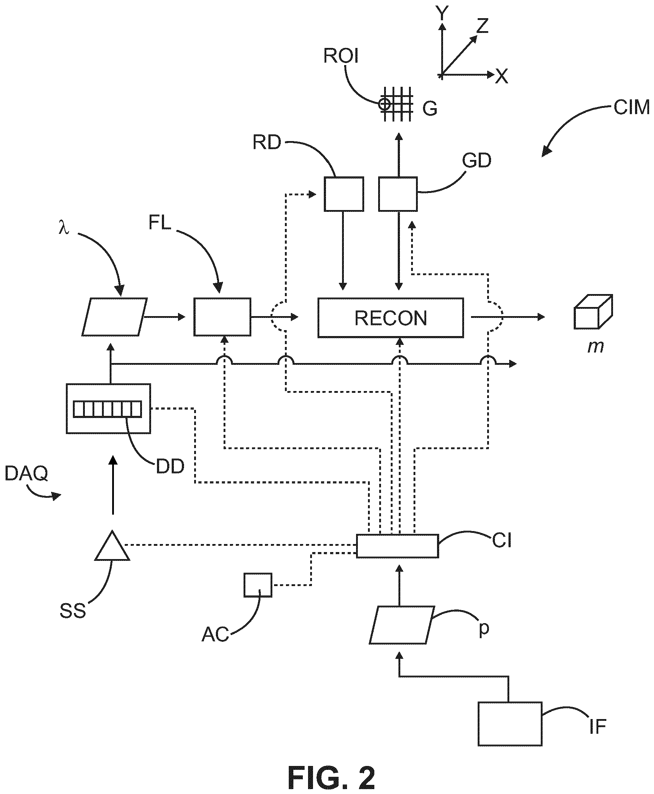

Overall operation of the imagery is through an operator console OC, a computing system with circuity such as processor (chip, CPU, etc.), data memory/storage, I/O-interfacing, bus systems, etc., together operable by a medical user/staff (technician, doctor, radiologist, nurse, etc.). Imaging apparatus IA is a complex entity. Adjustment of possibly numerous imaging settings of a multitude of components CIM (see Fig. 2 below of more details) of imager IA may be required, as dictated by medical imaging protocol and/or medical purpose/task for which the imaging is to be conducted.

Broadly, a given imaging setting (which may be considered a type of machine setting) may be made up of one or more imaging parameters that relate to settings of various such components CIM of the data acquisition section DAQ, or to settings of mechanical components that define the imaging geometry, or to any other component circuitry of the imager IA, etc. It is at least in parts the imaging geometry that determines the fields of view (FOV) of the imager IA. Imaging geometry may also include collimators, if any. Machine/imaging settings, as will be explained in more detail below, are automatically provided via the imaging facilitator IF in some embodiments.

Referring for now in more detail to the imaging settings, such may be understood to define/control behavior, configuration, operational features, etc., of the various imaging components CIM that together make up the imaging apparatus IA. For example, components of data acquisition section DAQ generally relate to signal acquisition. Components in relation to imaging geometry relate to definition of the spatial configurations between the ROI (the part of the patient PAT to be imaged), and one or both of i) the signal source SS, and ii) the detector device DD. Imaging geometry defining components may include electro-mechanical machine parts, and related actuators AC that together act to realize different such imaging geometries by effecting motion (deformation, translation, rotation, or a combination of any two or more ore all of the foregoing) on such one or more machine parts with actuators acting thereon suitably. For example, such settings may relate to the opening (its size and/or shape) of the collimator, if any, thus forming the beam in X-ray based imaging. More generally, the imaging geometry may be adjusted by imaging settings that control a spatial configuration of the source and/or the detector relative to ROI. For example, in X-ray based imaging, changing various angles α of source SS relative to a reference direction may determine propagation direction α of the X-ray beam through space towards the ROI. This in turn may determine the spatial information as encodable by the acquired projection data λ.

Examples of the above components CIM may include: whole or parts of gantry GT, such as a movable gantry/arm component GT in CT, or in therapeutic C-arm imagers as mainly envisaged herein. Gantry motions may allow angulations /tilts of source SS and/or detector DD. Other examples include one or more motions in relation to table PS (if any), such as sliding or tilting thereof, relative linear translation through imager's bore, whilst gantry is rotating about longitudinal axis of patient on table, such as in helical scan, etc. On occasion, table PS remains stationary, but it is the gantry with the table PS (and thus patient) in its bore that is so translated past table.

However, higher generation of CT scanners are not excluded herein, where multi-directional data acquisition is not effected by mechanical rotation of source SS on gantry GT, but instead by switching multiple sources arranged in an annulus around the examination region/bore. In MRI, there are no such motions other than perhaps moving table with patient on it into bore. Instead, in MRI, multi-directional data acquisition is effected by switching into transmit/receive mode a set of RF coils arranged in different spatial directions. The coils are able to pick up measurements λ as resonance signals, which signals may then be combined by MRI-reconstruction algorithms into sectional imagery/volumes of the ROI.

The above is merely a non-exhaustive illustration of operation of some components in imagers IA of various modalities. Other components in such or other imaging modalities are also envisaged herein, such as in nuclear imaging, US, etc.

In sum, each one of imaging geometry defining components and/or components of the data acquisition system DAQ can be adjusted by providing suitable imaging settings. Ordinarily, this may need to be done manually by the informed user. Here, however, it is proposed to assist the user to automatically, or at least semi-automatically, effect such imaging settings, merely based on a natural language imaging request put through by the user, as will be explored herein in more detail.

An imaging facilitator IF (to be described hereinunder in more detail) computes, based in an imaging request, a corresponding imaging setting for the imager IA at hand, and so readies the imager IA for the specified imaging task as per the request.

The present disclosure is mainly drawn to image guided procedures (IGP), such as interventional imaging (IR - interventional radiology). Such IGPs can be diagnostic or therapeutic, or partly both, or may serve any other purposes such as in medical or outside the medical field. In IGP, the imaging apparatus IA ("imager" for short) is particularly configured for imaging procedures with frequent adjustments to imaging geometry, which requires adjusting parameters of imaging geometry settings. In image guided procedures, a procedure is performed on a subject, such patient PAT, under image guidance, for the purpose of therapy, diagnosis, planning, or any other medical objective. In such procedures, at times, a C- or U-arm type imager is used, having a C or toppled U-shape gantry. In such imagers IA, the various imaging components such as signal source SS and detector DD are arranged in a gantry GT that has a recess, hence the C or U shape, that allows user ("interventionalist") access to the patient, even during an ongoing imaging session in which imagery is acquired. The signal source SS may be an X-ray tube, and the detector device DD an X-ray sensitive detector, and for such they will be referred to herein, cognizant of the broader principles envisaged herein, where the imager IA may be instead an ultrasound ("US") imager, or may be of any other modality suitable for IGPs of any sort. Thus, the procedure, such as intervention of any kind, may be performed in respect of subject PAT, and such IGP is image guided in that the imager IA provides a time series or stream of live action video imagery *m=(mₜ).* Individual frames *mₜ* at respective acquisition time *t* may be referred to herein as frame(s), and such feed, comprising such as time series of frames *mₜ* over a period *T* of time, *t*∈*T,* may also be referred to as a "run" or "series". Such stream of imagery *mₜ* may be visualized, rendered for view on a screen of display device DID as video feed. This visualization of time series frames (*mₜ)* may represent to user the dynamics of a physiology or anatomy of interest, such as blood vessels of the myocardium moving due to cardiac activity, or other dynamic settings that involve motion. In addition, or instead, certain implements, devices or tools, such as catheter, guidewires, etc., that are used in the intervention, may likewise be so in-image represented, when residing in the field of view (FOV) at the time respective frame(s) are acquired. Such live action video (feed) *m=(mₜ)* may allow user for instance to navigate to a lesioned site and perform the procedure.

The procedure may be performed in a sequence of different phases, with different sub-procedures being performed in some or each, and where different imaging settings may be required per phase and/or sub-procedure. Such image guided procedures may include cardiac procedures such as angioplasty, thrombectomy, or cerebral interventions, or others. For example, in angioplasty the lesioned site may be a stenosis (a stricture) in a vessel, which needs widening to improve blood flow there. The phases may comprise a navigation phase where a ballon catheter is moved under image-guidance to the lesion, followed by a deployment phase where the ballon is operated to expand the stenosis, also done under image guidance. Image settings for imagery acquired in each phase may be different. Imaging settings may be in need for readjustment in some or each phase, as different imaging objectives are pursued in each phase: different views may be needed in different phases to ensure best outcome, etc. Whilst the proposed set-up is indeed mainly intended for medical image applications, the principles described herein are not so confined and can also be applied in other settings outside medical, such as in IGP for the examination and repair/maintenance of otherwise inaccessible plumbing, hydraulic, machine, or cave systems, for example, or any other. However, the below will refer to medical applications in the main.

The imaging apparatus, in particular of the interventional type, allows dynamic adaptations of its imaging geometry to acquire frames or runs *m=(mₜ)* at different views at different times. For example, this allows the user during the procedure in the different phases to change the imaging geometry to obtain a different view from a different spatial direction on the region of interest such as the lesion or the vessel system in which the user is navigating a catheter guidewire. The live action imagery is preferably in the projection domain so are projection images. However, 3D reconstructions from projection imagery in imaging domain using tomographic algorithms are also envisaged herein, as is spectral (multi-energy imaging) that allows material specific contrast. Thus, in some such cases, projection imagery λ may be processed into other type (such as spectral) projection data λ'. Such processing may be implemented computationally by an image processor IP or any other. Thus, the series of images that confer different views on the region of interest may be projection domain only, or may be tomographic volumes only, or may be a mix thereof as needed for a particular phase of the procedure. In addition, or instead, other processing may be applied such as in spectral imaging. Thus, in general depending on the processing, the output "source" imagery *m=(mₜ),* whether not processed by optional source image processor IP, may be made available via the imaging system MIA's imaging interface IIF, either wireless, wired, or partly both. It is this source imager (frames) *m=(mₜ)* that are used herein for supporting the IGP, and the proposed imaging facilitator system IF is configured herein to facilitate obtaining such source imagery *m=(mₜ)* at certain specially modulated IQs, as will be described in more detail hereinunder.

Turning now in more detail to imaging geometry, in general, changes of the imaging geometry throughout the imaging session in IGP may be affected by the mentioned actuators AC operable on request by the user through suitable user interfacing, such as button, joystick, foot pedal, etc. Imaging geometry in general refers to the spatial configuration or constellation of the X-ray source SS, the detector DD, and the region of interest in respect of which the IGP is performed. Such imaging geometry can be described by pose (position and/or orientation) in 3D space of an imaginary imaging line that can be to run from the focal spot of the X-ray tube SS to a center point on the radiation sensitive surface of the X-ray detector XD. In such a dynamic set-up as envisaged herein, it may be challenging to switch all aspects of imaging geometry and supply the correct imaging setting to effect such changes to the various components CIM, in particular in high stress / high workload environment as is often the case in the medical field, such as in trauma rooms, "cath lab", or other, in particular in large medical facilities.

To this need, the facilitator system set-up IF envisaged herein facilitates such, possible frequent and/or are rapid changes, in imaging settings. The imaging settings may be computed in quasi real time. Such may be displayed to the user on the image display device DID, or on another display device, different from the display device DID on which imagery is displayed. For example, the imaging settings may be displayed at a dedicated display unit of operator console, or other imaging "cockpit", thereby guiding user on which imaging settings to use, or how. Alternatively, or in addition, the imaging setting may be directly applied to suitable control circuitry interface CI to effect the imaging settings, such as imaging geometry changes in any one or more of a rapid, robust, and safe manner. In particular, the facilitator system IF will be described in more detail below, however reference is now made first to Fig. 2, which provides more detail on the imaging set-up generally, and, in particular imaging settings, and imaging parameters to which they pertain, that may need to be adjusted, depending on imaging context, which may be likewise subject to change. Such context may include the particular imaging protocol, the IGP, its phase, the current sub-procedure, etc.

Image setting may describe a state, setup, configuration, etc., of one or more, or all components, as described above in Fig. 2. Each such imaging setting can be described by one or more numbers, codes, etc., collectively referable herein as imaging parameters. Thus, each imaging setting may correspond to a particular combination of imaging parameters, and conversely each such combination may correspond to an imaging setting. Thus, one determines the other, and "imaging setting" vs "imaging parameters" can be used herein interchangeably.

In more detail, Fig. 2 which shows the manifold components CIR that need to be provided with imaging settings *p* (such as instructions. etc.). Imaging facilitator IF provides such, settings *p.* These are propagated through control interface circuitry CI, including suitable interfacing API's, to the various components CIM of the medical imaging apparatus IA. The imaging controlling components CIM of imager IA may include the signal source SS, the detector DD. More generally, such components may be members of the data acquisition unit DAQ operable to acquire the measurements λ, λ'. In addition, downstream to acquisition, the acquired measurements may be processed, such as by a reconstruction algorithm RECON, to compute tomographic or volumetric imagery *m* in image domain. However, in solely projection-based imaging such as radiography, output imagery may be still in projection domain. Thus, in some embodiments, the output imagery *m* is, or least includes, the measurement data λ.

The settings may also relate to certain parameters of a filter component FL, or indeed to parameters such as regularizer parameters, or other parameters of the reconstruction algorithm RECON or filter component FL to be used.

For example, such tomographic reconstruction algorithms RECON may include filtered back-projection (FBP). iterative, algebraic, or machine learning based as needed.

The region of interest definer RD relates to an imaging geometry defining component. As mentioned, such may effect mechanical movement of the gantry for example, and thus of the source SS and/or the detector DD, and/or may effect motion control of the patient support PS, such as in CT or magnetic resonance imaging. Another imaging geometry defining component may include the collimator in X-ray. Thus, some parameter(s) *pₘ* may relate to positions of radiopaque collimator blades of collimator.

The grid definer GD defines the mentioned spacing of the spatial grid and voxels in an image co-ordinate system of the examination region (*X,Y,Z*)*.* The resolution for example may be set in this manner, based on a suitable parameter of the imaging instruction.

In addition, the imaging instruction *pₘ* may also include parameters for the visualizer VIZ, such as to set level and windowing parameters to optimize the manner of displaying the image values. Level/windowing describes the mapping of image values in imagery *m* to a range (a "palette") of color or grey values. For example, CT imagery m may have a vast dynamic image value range, and displaying such imagery at wrong settings may drown out details that may be crucial for the imaging purpose at hand.

With continued, and with yet more detailed, reference to Fig. 2, this is a schematic representation of imaging pipeline as may be used herein, for C-arm, CT, or MRI, or other. Thus, the block diagram of the pipeline illustrates various components CIM, some of which may be optional. Some or each of the components may be controlled by respective imaging settings/parameters *pj,* which together form the imaging setting p.

The projection data λ as measured at detector DD along different projection directions α^{d} is receivable at a projection data input port (not shown) of the pipeline. The received projection data is optionally processed by a filter component FL. The filter component FL may be operable as a band pass filter, a low pass filter or a high pass filter, or any other. The filter component FL may be operable as a noise filter to reduce noise in the measured projection data λ. A noise model may be used such as statistical or any other.

The optionally filtered projection data is processed by a reconstruction component RECON into reconstructed imagery in image domain G. The reconstruction component RECON may implement a reconstruction algorithm.

A reconstructor component RECON may implement one or more reconstruction algorithms to process the projection imagery into imagery in image domain, such as the preview imagery or the final image. Specifically, the reconstructor RECON may compute the output image *m* as sectional imagery of the examination region (with the patient in it) for diagnostic, therapeutic or other purposes. The reconstructor RECON may be able to produce sectional volumetric image data m. However, this does not exclude producing a single image slice in the examination region as required. Thus, the reconstructed imagery may include the whole volume ER, a partial volume thereof, or a specific section therethrough. Volumetric reconstruction may be facilitated by helical movement, and/or the 2D layout, of the X-ray detector DD.

Any suitable reconstruction algorithm may be used by reconstructor RECON component, such as algebraic, statistical or analytical. Analytical reconstruction algorithm may include filtered back-projection (FBP). Iterative reconstruction algorithms are also envisaged in preferred embodiments.

Any one of the above mentioned components, such as the filter component FL, the ROI definer RD, the grid definer GD and the reconstruction component RECON may be arranged as separate, discrete, components as shown in Fig. 2. However, this may not necessarily be the case in all embodiments, as the above, "spread-out", layout in Fig. 2 is largely driven by clarity of presentation. Thus, in other embodiments, some or all of the components may be coalesced into fewer components by integration or combination, or even into one single component. For example, the filter element component FL is sometimes part of the reconstruction algorithm implemented by the reconstruction component RECON.

Operation of each or some of the said pipeline components CIM may be a function of the said imaging setting *p.*

The facilitator system IF may pass settings p to a control interface CI through which different such machine settings p may be fed into the pipeline RP. Thus, the facilitator IF allow controlling operational behavior of the pipeline. Thus, the interface CI populates memory placeholder (free parameters) related to the various components, with the received parameters p, and so sets them to certain values, thus controlling operational behavior of the pipeline.

Such parameters that make up the imaging setting may include parameters of the noise model and/or of the reconstruction algorithm.

Yet other parameters p of machine setting may include one or more parameters of a noise model, one or more parameters of a reconstruction pipeline, in particular, of the reconstructor RECON in itself such as certain parameters any one of parameters of the reconstruction algorithm, and as said, the size of the grid, the number of projection data frames to be used, the size of the ROI to be reconstructed in image domain, the number of iteration cycles, etc. Other parameters for the parameter setting may include regularization parameters, strength modulators such as me be configured in a denoising /noise suppression algorithm of the filter FL, or in the iterative reconstruction algorithm used by reconstructor RECON.

In MRI, the parameters *pₘ* may include pulse sequence definitions, that is, a list of instructions of which of the RF coils *Cⱼ* (*Cₓ C_{y}, C_{z}*) to energize, by how much, for how long, and when, in which order, etc. Other parameters may relate to MRI reconstruction implemented by reconstructor RECON, such as operations in k-space, Fourier filter coefficients, manner of read out from k-space, and may others.

As further illustrated in Fig. 2, and in particular in relation to dynamic X-ray image guided procedures ("IGP"), and the use of imager IA for such, the components CIM may include one or more actuators AC that allow changing the imaging geometry, in particular changing the imaging axis, as described above in reference to Fig. 2.

In particular, a position/orientation of the signal source SS such as the X-ray tube, and/or of the detector DD can be changed, and so can a collimator setting, for example. In addition, the energizing of the X-ray source can be adjusted by applying different tube settings such as voltage and/or amperage, for example, the tube that describes the voltage across the tube's cathode and anode. Another X-ray setting may include focal spot size, and/or pulse time etc. In addition, or instead, the amperage of the anode may be changed that controls how many electrons are released, whilst the tube voltage determines the acceleration and thus the energy of the X-ray beam issuing forth from the source, in particular of the focal spot. With different views this may require different changes as the through-tissue thickness (tissue path length) across views may change depending on the anatomy on the consideration, in particular depending on the region of interest.

The components CIM to be set and controlled by appropriate imaging settings may further includes a contrast agent delivery device (such as a pump - not shown) to deliver the contrast agent, thus boosting contrast for natively radiation transparent structures, such as vessels in angiography, as mentioned earlier.

It will be appreciated that the above list of parameters and components CIM are merely illustrative and non-exhaustive, and, depending on the imaging task, some components may be used, whilst others are not. Thus, some of the components above are optional, such as reconstructor, the contrast agent delivery apparatus, etc.

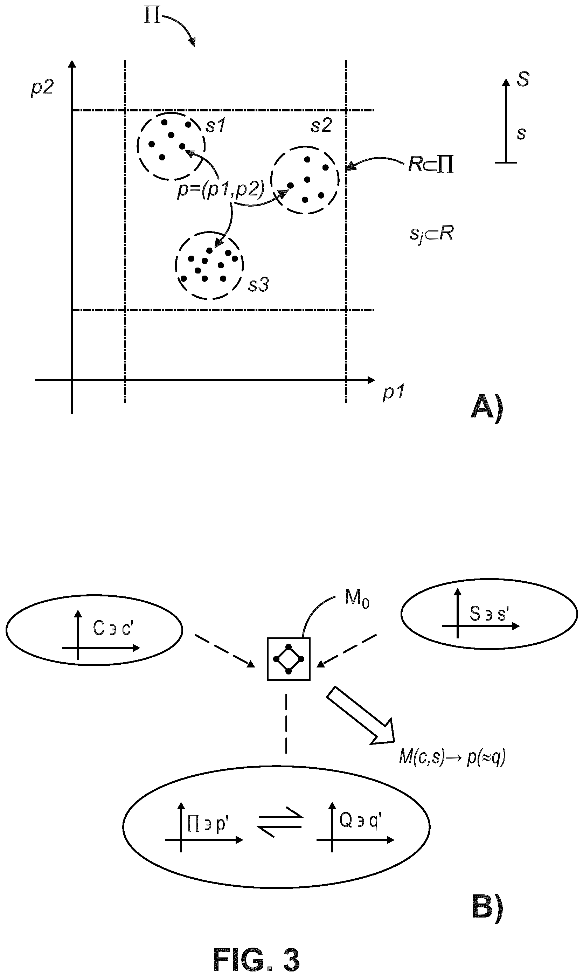

Having thus described various imaging settings with reference to Figs 1 and 2, reference is now made to Fig. 3 which provides more details, in a conceptualized fashion, on the operation of the facilitator system IF.

The described imaging settings *p* may be identified with parameters that determine the imaging settings of the various machine components CIM. Such imaging parameters or combinations thereof, for one or some or all imaging components CIM may be concatenated, and described as elements of a vector space with its co-ordinate pertaining to different such settings, in particular different imaging parameters *p = (p1, p2, p3, ... pN), N>=1* of various imaging settings of a given one or more components CIM, as described above in Fig. 2. For example, one dimensional coordinate *p1* of this, in general high dimensional vector may pertain to tube voltage, another co-ordinate *p2* may pertain to the tube amperage, yet another (not shown) to blade positions of collimator, and so on and so forth, for some or all of the above-described imaging settings in Fig. 2 and their related parameters. The view in Fig. 2 is highly simplified and schematic in that the imaging parameter vectors *P* are shown in a mere two dimensional co-ordinate system (*p1, p2)* in a low 2D space, but there may be in general a much higher dimension, in which dimension *N* may run into 10s or 100s of spatial dimensions in such as data space, referred herein as the "imaging parameters space" Π ∋ *̅p̅*̅ =: *p*. Each imaging setting P can be seen to thus correspond to a point in such a vector space, such as in a (Euclidean) affine vector space which entertains notions and configuration for distance, length etc., between vectors, points, etc. The imaging settings from all or some components CIM can be consolidated into a single vector, or can be grouped in different vectors. In the latter case, rather than corresponding to a point, the imaging settings correspond to a point cloud.

The imaging settings described in Fig. 2 above are assumed to be herein available in a suitable numeric form which may be so natively but may not necessarily always be so and may need to be transformed accordingly by an embedder into a suitable numerical form, such as the components *pj* of the said vector(s) *P.* The term imaging setting "point(s)" or "vector(s)" will be used for simplicity interchangeably herein, using whichever is more natural and apt in the circumstance, with the understanding that each point can be viewed as a vector and vice versa, thanks to affine vector space properties. In more detail, the embedder may use embedding algorithm. Such algorithm may be used that express or transform imaging setting data into respective vectors in high dimension vector space *Π.* One or many hot encoding schemes may be used for instance. Via embedding, the mentioned vector space distance function (such as Euclidean, or other) may be used to define/compute distance between different settings as points/vectors or in the vector space. The embedder may be part of a functionality referred to herein as a data preparation module (not shown).

The imaging setting vectors when represented, stored or processed in such a parameter space Π are not randomly distributed it has been found, but their distribution may follow a certain (spatial) pattern in data space *Π.* For example, in medical imaging it is the object to achieve certain image quality ("IQ") of frames *(mₜ)* that form the video feed *m.* The distribution patten corresponds to the respective context (IGP phase and/or sub-procedure) that the respective frames are to support (that is, to "guide"). Broadly, IQ describes properties of the images to be obtained. It is apparent that the expenditures (such as dose in X-ray) needed to acquire imagery at a needed IQ has an impact on patient and/or staff, particularly in an interventional setting, and many other aspects. When sampling various imaging settings from user across multiple use cases, or from plural users, imaging departments, clinics, or whichever such user entity, there will generally be some broad consensus among the medical profession what imaging settings to use for a given imaging task or IGP. Thus, distribution of such imaging settings can be expected to broadly fall into a feasibility range *R*⊂Π. This is an *N*-dim set in which such imaging settings point parameters ought to lie for a given imaging procedure. Characteristics of such a feasibility region *R* is usually a function of the particular imaging protocols, etc., itself a function of contextual data, such as any one or more of clinical purpose of the imaging, the IGP, its phase, patient bio-characteristics, medical history, the region of interest ("ROI") in respect of which the IGP is performed, etc. However, it has been observed that the feasibility region R is not generally clear cut in that it is not a point in space as one may have thought, but is instead, just that, an N-dim region. Thus, there is a certain variation in the imaging parameters for any given imaging task or procedure, as used by user entity across cases they handled, or across different user entities and their cases. Thus, although the accepted, preferred, etc., imaging settings broadly aim for the same or broadly similar image quality, it has been found they may still allow for a certain variation among different users. But on closer inspection, the N-dim (N-dimensional) feasibility region, has yet another structure, as may be understood with further reference to Fig. 3A. There, the broad feasibility range *R* is displayed in the parameter space Π as a hyper-(N-)dimensional cuboid, which, as a whole, represents the generally agreed objective image quality and their related settings/parameters that enable such IQ. The region R may have a clustered microstructure. Thus, for clinically relevant image qualities for a given context *c,* the parameters are generally thought to reside in such respective cuboid R '(or any neighborhood/set/data region of whichever shape) that represents general medical knowledge. Such knowledge may follow the ALARA principle ("As Low As Reasonably Achievable"), as pertains in particular to dose usage in X-ray imaging. The imaging parameters in a given data region *R* for a given task/procedure/phase, may represent certain image "flavors", as may be preferred by a given user in similar tasks/procedures/phases, or by one user vs another user. Thus, the different "flavors" or preferences for imaging settings, and hence for IQ, of a user may be conceptualized as more or less distinct clusters *Sj* such as *S*_{1, 2, 3}, *j=1... 3* for a given context c. Merely three such clusters are shown in the example of Fig. 3A, but there may be less or more of such clusters. Each such cluster *Sj* can be mapped or parameterized, *s-> Sj,* with a certain image score *s* along a personalized scale *S.* The scale S could be a discrete scale as shown, that proceeds in whole numbers, such as from *1* to *10,* or the like. For example, a binary scale *"0","1"* may be used, Alternatively, the scale can be a continuous scale from zero to 1 for example, as needed. Thus, score s could be whole number, or a fraction.

The facilitator system IF as envisaged herein not only allows setting automatically the imaging parameters/ imaging settings *p,* which ought to be applied for a certain procedure, or a certain phase in a given procedure, but is configured to take into account the user's preference *s* in terms of image quality. Thus, what is proposed herein is a facilitator system IF that takes as input an indication of the procedure/phase to be performed which may be understood as a general contextual parameter c and, in addition, and over and above such parameter c for context, there is an indication for the personal image quality *s* sought, parameterized by the score s over scale S. The score may be explicitly or implicitly provided as input to (imaging) facilitator system IF by user via user interface UI. However, such score is in general pre-set for the given user and does not need adapting. In normal operation, the score will be set for "optimal image quality". The preset score may be explicitly overruled as input to (imaging) facilitator system IF by user via user interface UI, such as when user wishes to intentionally deviate from this score, as the user may believe the training data on which system's ML model was trained for is in not adequate for the current imaging task. For example, user may want better IQ, whilst dose being of lesser consideration for the specific task, phase, instant, etc. at hand at that moment in time.

Thus, input to facilitator system IF is in general multichannel *(c,s)*, one channel for context, the other for personal/preferred IQ score s. What is more, that score s may be per the whole IGP, or may be atomized into "atom scores", any or more such atom score per phase or sub-procedure. Specifically, context data c may specify the procedure, sub-procedure phase, etc. Thus, score *s* may be dynamically changed over time by user via interactive UI, and each such user requested change may trigger new imaging settings to be applied. Thus, score may may have a spatial dimension along scale S, and may further have a time dimension as per the IGP proceeding over plural phase, sub-procedures, etc. But for main purposes envisaged herein, the score s may remain constant for the given imaging session, and it is merely parts of the context c that is being changed/updated. The subjective score s follows in general the ALARA paradigm: good enough for the clinical task" (good enough IQ, not too high X-ray dose). New imaging settings may be applied frequently due to constant changes in the contextual data c.

Thus, imager IA may be operable to acquire imagery, not only at different imaging settings, but at different personalized preference that varies, as score s is varied by user, within a given, ongoing IGP, or among different IGPs, as needed. The facilitator system IF facilities imaging by allowing user, in a no hassle setting, quickly find and apply their preferred flavor of a given IQ.

In more detail, such score s may be conceptualized to represent in general the clusters of the respective imaging parameters that give rise to a broadly image quality as per a given protocol that specifies ranges of its imaging settings/parameters *p=*(*pj).* Thus, personalized image quality s can thus be conceptualized as such different clusters in the parameter space Π. Alternatively, or in addition, such personal imaging settings can be identified as different conditional probability distributions over a population of such imaging parameters, as can be reconstructed in approximation based on historical imaging settings as may be collected across various sites, or across various individuals from different sites etc., or from a given user over time. The probability distributions may be defined in terms of a respective conditional density *f(p*|*s),* which is concentrated at different portions of space R, namely over the respective cluster. Thus, personalized image quality, via their associated imaging settings *pj,* can be objectively defined with recourse to such clustering or probability distributions, given a suitably varied set of such parameters as may have been applied in the past, and as may be found in medical imaging databases, such as a PACS of a hospital, or as may be drawn from various hospitals or other medical facilities.

The facilitator system IF is capable of modelling the relationship between a triad of data: the contextual data C, the scores *S*, and the imaging settings *p* which are associable with a certain objective image quality *Q.* Thus, *p* ∈Π implicitly defines *q* ∈ *Q*. Thus, this triadic relationship is illustrated at the bottom of Fig. 3 at schematic diagram Fig. 3B. Modelling such a relationship analytically may be difficult, which is why machine learning ("ML") models/algorithms are preferred herein, that do not require an explicit analytic set-up to capture this relationship. Instead, provided enough data of suitable variation, this relationship can be learned as patterns from certain ML models such as neural networks, in particular of the convolutional type, or others, that require merely a very relaxed modelling assumptions. Such model M*^{θ}* can have its model parameters *θ* (different from the imaging parameters *pⱼ*) adjusted in a training procedure to obtain a trained ML model *M* that can deliver, post training, this personalized imaging setting as illustrated in the right hand of Fig. 3B. Training may be a one off, but can be repeated, once enough new data is available. In such case, the model trained in an earlier cycle on earlier training data may be supplied as pre-trained model, is this than trained again in a new cycle on such training data, based on the pre-trained model as a starting, initial model, and so forth over potentially plural generations of models subjected of different training cycles.

In Fig. 3B the prime " ' " symbology indicates existing training data, such as scores *S*' earlier awarded/used, related historical contextual data *c'* , and the imaging settings P' which were used such historical cases, each such P* associable with a certain objective image quality as can be measured by image metrics, such as SNR, and others. Thus, the objective image quality parameters *q*' are different from the subjective scores s which represent the different distribution in parameter space of the imaging parameters *P* as shown in Fig. 3A. In training, an initial machine learning model *M0* initialized with random or uniform model parameters *θ*, or a pre-trained model from an earlier training cycle, is applied to such training data. The training data may include historical user derive or expert derived scores s', context c' to compute therefrom the intended imaging parameter p' that fits to such a score and context *C.* Thus, the training procedure yields, as shown by the heavy arrow to the right of Fig. 3B, the trained model M. The trained model can be conceptualized as a mapping *M: (c,s) ->p* that maps image context C and the user requested quality score *s* (the personalized score *s)* to the imaging parameter *P,* which may correspond to an objective image quality *q* in the feasibility range *R.*

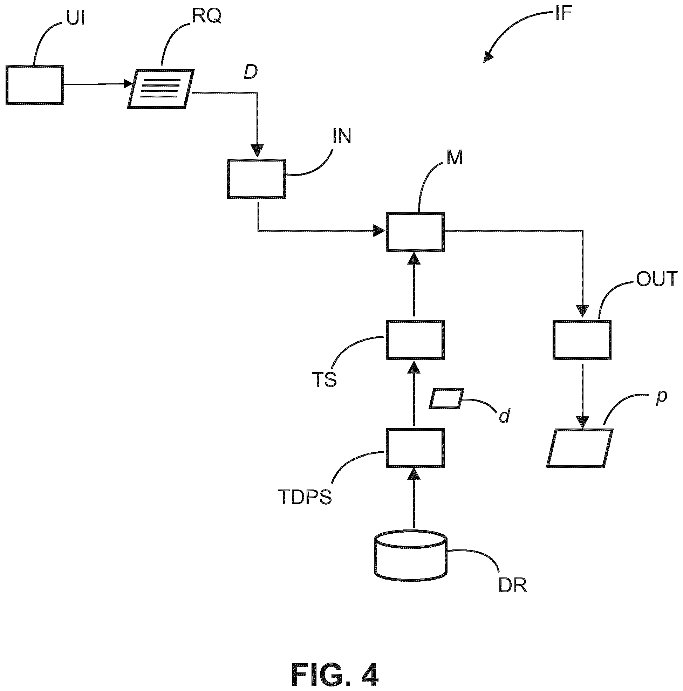

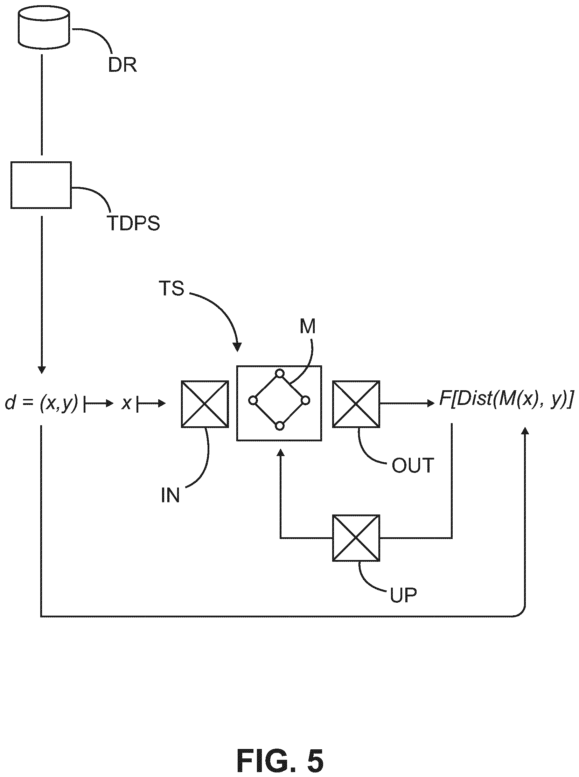

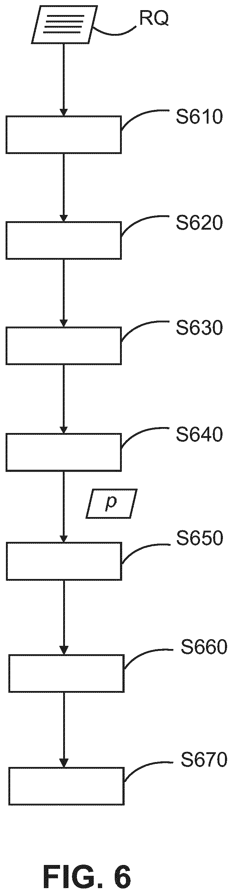

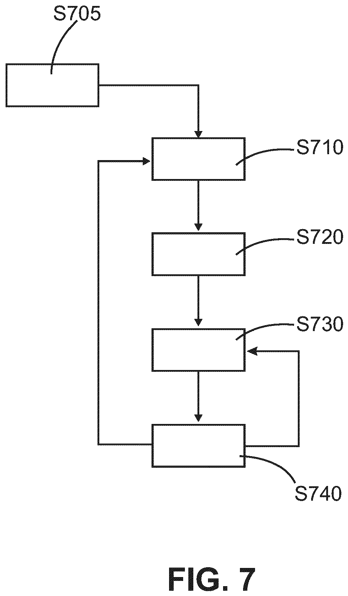

In the following, operation of an ML based facilitator system IF will be explained in more detail below, assuming that it has been suitably trained based on the principles described in Fig. 3. Training aspects will be discussed in Figs 5 and 7. Specifically, in Figs 4 and 6 it is assumed that the model M has been trained suitably already, along the lines discussed in Fig. 3, whilst the training itself will be described in Figs 5 and 7.

Turning now first to the block diagram of Fig. 4, operation in deployment (after training) of the imaging facilitator system IS will now be described. An imaging request *RQ* is received at the input port of the imaging facilitator IF. The imaging request mainly relates to the context data c. The imaging request may be supplied by user interface, such as by indication in a graphical user interface, by speech recognition, by gesturing, and/or in whichever form, as needed and appropriate. The request may be for an intended imaging operation such as part of an IGP, or other purpose, but it mainly envisage herein for all manners of IGP. The imaging request may be for the imaging operation of imaging apparatus IA, such as a C-arm system or other. It may be for the imaging operation as a whole, or for a certain phase thereof, a sub-procedure or IGP, or other. Such multiple such requests may be received in sequence, one or more for some or each phase of the imaging operation as it unfolds, such as in the said IGP.

The imaging request includes in particular the imaging context *c* and, in addition, includes the personalized image quality score *s* sought by the user during the current imaging procedure. The context may change from request to request. The context may specify patient data, the view intended in medical terms, the purpose of the imaging. Some aspects of the context may not need to be respecified for each new request, such as patient data, for each request in a given procedure, as same aspects may remain the same-

The request (data) *R* is received at input port IN, and is then processed by the trained machine learning model M into the imaging setting *p* which is then output at output interface OUT. The imaging setting can then be displayed or otherwise, such as by sounding out, etc., and/or can be (directly) passed to the control circuitry interface CI. The control circuitry interface CI translates the imaging setting *p* into corresponding commands, control signals and like, that are applied to the one or more associated components CIM, thereby causing the imaging settings to be applied to the imager IA, thereby resetting the imager IA. The image acquisition may be automatically triggered on application of the new imaging setting *p,* or it is user who triggers same after taking note of the computed setting *p.* The imaging settings *p* may be applied automatically, or by user, for example on approval by the proposed settings *p.* Thus, the settings *p* may be displayed first to user, or otherwise brough to user's attention. For example, after a review/consideration of the new imaging setting *P* proposed by ML model M, the user may operate control interface at console OC to apply the settings and cause image/frame acquisition at the new setting, and so one for any new request. For example, the imaging setting *P* computed by model M may be output through output interface OUT to be displayed on user display device DID. The display device for display of imaging setting P may be different from the display device DID used to display the imagery *m,* or the same display device DID is used for both. But as said, computed setting P may be displayed on a dedicated control display of the operator console OC, as needed. In addition, or instead of displaying, the new imaging setting *p* are merely sounded out in natural language by an NL module (not shown), or are provided in whichever manner suitable, user-convenient, and conducive to the clinical task at hand.

Referring now back in more detail to the request (data) R, this comprises input data D received at input port IN, and as supplied by the user. The input data D includes imaging context data c, and the personalized image quality score s as described above in Fig. 3. Thus, input D as processed by model M can be formalized as multi-channel input *D=(c, s).* Just like the imaging settings *p,* the input D data can be embedded as vectors in a suitable dimensional vector space. As said, if the data is not in numerical form, it can be processed at the input interface by suitable data preparing, such as data coding, in particular by using one-hot- or multiple-hot encoding or other. Such data preparation encoding can be done beforehand as needed by using suitable encoder and/ or embedder, as the case may be, and it is assumed herein that such input is represented as a point in a high dimensional vector space. Vectors *D=(c, s) = (c₁, c₂, ... cₘ, s)* can be formalized herein as elements of a Cartesian product space (*Π* × *S*). The *cⱼ*'s are component coordinates of the context part of the vector *(c₁, c₂,* ... *cₘ, s)* and may represent different aspects of imaging context, such as patient data, imaging purpose, etc. Some of this data may be categorical data. One-hot encoding could be used. ML itself may be used to learn suitable vector representations for *P* and *D=(c, s).* For example, in data preparation, *a*n auto-encoder architecture could be used to learn, either or *D=(c,* s) or *p,* as the latent representation as could be read-out from a hidden layer of the auto-encoder, after auto-encoder is trained to recover input (*p* or *D=(c, s))* at its output.

The provision of such input data *D* to the trained machine learning model M for computing by the model M, based in *D,* the applicable imaging setting *p* may be a one-off, or may be done repeatedly with different input data D. Thus, facilitator IF may be operable in a dynamic procedure, in particular when used, as envisaged herein in main embodiments, in IGPs where, for example, frequent imaging geometry changeover and/or tube SS re-settings may be called for. Thus, as the image guided procedure progresses through its various phases, in which different tools (guidewire, sheaths catheters, flow diverter, stent, etc.) and/or different field of views may be called for, the user can put in new input requests at commencement of such different phases. The model M of imaging facilitator IF will then respond dynamically, preferably in real-time. Thus, model M computes, based on new input data, new updated imaging setting. This can be done plural times over, such as two or more times, until the IGP terminates, and as needed and deemed fit by user. Thus, the outcome is that, given current input data, a certain run of frames is acquired at the computed parameter *pₜ,* whilst the next run of frame(s) is acquired at new imaging setting *p*_{*t*+1}, based on new input data Dₜ₊₁ as may may be supplied by user when IGP enters a new phase of the procedure. With the new input data, the user may control the output *p_{t,t+1}* by modulating behavior of model M, by retaining the same personalized image score, or by changing same.

Thus, in operation, the output of facilitator ID in context as such image guided procedure is a time series of imaging settings *pₜ,* possibly in correspondence to different phases as the imaging guided procedure unfolds. Thus, at one point in the procedure, new input data is applied. The imaging setting is re-computed in response to new input data. The re-computed imaging setting is then used for the next frame(s) in the live action video feed as may be displayed on the screen DID. Thus, the imaging settings *pₜ* can be thought to be updated in correspondence to the input data *Dₜ* as applied by the user during the procedure.

As said, the input data *D* may be provided by suitable user interface UI, including speech, gesture recognition or any other. At times it may happen that the user is not satisfied with the image quality obtained at a given time, at which point user may be able to over-ride the proposed imaging settings. Thus, user may choose to use the current imaging setting as proposed, or user may change the proposed setting manually, and re-acquire the imagery at the changed imaging setting. Thus, model will update the imaging settings in response to a "not satisfied" user input. For example, the user may simply specify he wishes to have a higher or lower personal preference image quality s, and the system IF then applies the current context data settings anew, but in combination with new score s, to compute a new image at a slightly different IQ flavor. In this manner the system supports the user "fine-tuning", in terms of quality flavors or other feedback, the imaging settings.

In order to better support the above dynamic aspect, in particular in IGPs, the system IF may be implemented on high performance hardware, such as multi-core chips sets, generic or specialized, graphics chips (such as GPUs), instead of, or in combination with, HBMs for rapid data and/or instruction loads. Calculation involved in the forward passing of input D though model M may be based on vector and/or matrix operations, such dot-producting, or matrix multiplication, in particular for NN type models. In this or other manners, a single-instruction-multiple-data ("SIMD") type parallelization maybe implemented.

### Training

Wirth continued reference to Fig. 4 and with reference to Fig. 5, the trained model M may be provided, in a training phase preceding test and deployment/inference phase, by a computing system, referred to herein as the ML training system TS, that implements an ML training algorithm. Broadly, the training algorithm adapts parameters of model based on training data *d=(c',s', p')* as may be held in one or more data repositories DR. Such training data d may be provided by a training data provider system TDPS, which may itself be a suitable computing system. Operation of training system TS and of training data provider system TDPS is now described.

Turning first to the training system TS, this is based on training algorithm and a model architecture for model M. In general, ML proceeds on two or, optional, three phase in the following order: training phase, then testing (generally with several iterations until satisfactory), and finally deployment.

In the training phase, model M is trained on data d. Training phase may involve multiple training cycles, including pre-training. Once trained, test data (different from training data) is used to evaluate performance of model. If error rates are acceptable, model is released for deployment, such as for use to serve intended purpose for which it was trained/tested for. In the instant setting, such use/deployment is to assist the medical user/professional in operating an imager in day to day clinical practice. The data to which model is exposed in deployment is in general different from training and test data. The expectation is that by the training process the model M has "learned" underlying patterns in the data that allows the model to suitable generalize when processing hitherto "unseen", new, data RQ, and still produce useful (reliable) results p, on which medical imaging user can act on in clinical practice.

It is an object of the training system TS to optimize, and hence adapt, the parameters *θ* based on the training data *d=*{(*x: =(c',s'), y: =p'*)}*.* In other words, the learning can be formulized mathematically as an optimization scheme where the said cost function *F* is minimized. although a dual formulation of maximizing a utility function may be used instead.

Assuming for now the paradigm of a cost function *F,* this measures the error incurred between data estimated by the model *M(x*) and the associated target y, as per some or all of the training data pairs *k:* ${argmin}_{θ} F = {\sum }_{k} Dist \left[{M}^{θ}\left({x}_{k}\right),{y}_{k}\right]$

In eq. (4) and below, function *M*() denotes the result of the model applied to training input *x.* As said, the cost function may be based on a distance function *Dist []*. The nature of the distance function will depend on the nature of the underlying model. The distance could be an 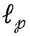 distance in vector space having an 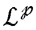 norm, with 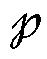 non-negative integer, such as p = 2 for Euclidean distance. This distance function may be useful for regression type models. For classifier models, cross-entropy or Kullback-Leiber divergence or similar may be used.

In general, there is an error between training output *M*(*xₖ*) and the associated target*yₖ* of the presently considered *k*-th training data pair. An optimization scheme such as backward/forward propagation or other gradient based methods may then be used to adapt the parameters *θ* of the model M so as to decrease the error for the considered pair (*xₖ, yₖ*) or for a subset of training pairs from the full training data set. The whole of the training data set may be used at once, or model proceeds in batches over subsets of training data. Each batch uses as the respective starting point the parameter learned from previous batch. In some instances, no summation is needed in (4).

After one or more iterations in a first, inner, loop in which the parameters *θ* of the model are updated by updater UP for the current batch (set) of training data (pairs) {(*x_{k,}yₖ*)}, the training system TS enters a second, an outer, loop where a next training data pair batch {*x^{k'}*, *y^{k'}*} is processed accordingly. The structure of updater UP depends on the optimization scheme used. For example, the inner loop as administered by updater UP may be implemented by one or more forward and backward passes in a forward/backpropagation algorithm. While adapting the parameters, the aggregated, for example summed, residues of all the training pairs in the current batch is considered, to improve the objective function.

The training system as shown in Fig. 5 can be considered for all learning schemes, in particular supervised schemes. Unsupervised, or self-supervised learning schemes may also be envisaged herein in alternative embodiments. GPUs or other CPUs with multicore design may be used to implement the training system TS, suitably parallelized.

Turning now in more detail to the training data provisioning itself, the training data provider system TDPS may include a viewing station, such as a computing device coupled to a display device (monitor, etc.), and may further including a user input interface, such as pointer or other annotation tool. As such, the viewing station may be same as used by radiologist viewing sessions. The room in which the review takes place of training data provisioning may be dim lit to aid review. The user, for whom the machine learning model is to be personalized as described above, can then review, in one or more sessions, a pool of historic or synthetically generated imagery. Thus, the imagery may visualized in a standard viewing setting on the display device of the viewing station. On such visual review, user can award, by annotation, a score to rank the image based on their subjective personal image quality preference. The user can do this by viewing the imagery one by one or in parallel, as the case may be, and individually assign to each reviewed image a respective score s' according to a pre-set scale, say from 0 to 10, or any other. This is then repeated until a sufficient number of images have been so rated according to the user's personalized score. Preferably, the imaging apparatus IA itself may be configured as training data provider system TDPS , where the annotation would happen during use cases. Especially for the fluoroscopic imaging, the image quality needed for hand eye-coordination can most easily be judged during the clinical case, and this allows using the fluoroscopy (angiograms themselves as a base for review as such imagery may not always be sent to PACS. If the imager IA is itself so used, it may include a user functionality, UI, a button, a UI widget, or in whichever way, through which user can supply their training score s'. This training data collection functionality can be run initially for a certain period of time until enough training data is accumulated. It may then be disabled. It may be re-enabled once further training is wanted.

Prior to viewing, during or after review, the imaging settings that were used to acquire the respectively reviewed images are accessed. This can be done by examining entries in the medical database, such as PACS, where the images were retrieved from or can sometimes be ascertained by grabber tool by investigating header data in which the imaging settings that were used for the respective image are stored. In this way, the training data input data for the imaging setting p' can be obtained. Similarly, imaging context data c', such as patient data or intended imagining purpose, can be likewise obtained from the medical record or from metadata, as the case may be. Thus, from this triadic pool of data, training data can be built up of the form (*c', s', p').* In such triples, the first entries *c*' and *s*' form the training data input for one instance, with its associated imaging setting p' being the associated target (*c*', *s')-> p'.* The order of the c' and the s' is immaterial. However, when feeding the training data into the model for training, the order or position in the training data (such as the entry position in a vector or matrix) should preferably be consistent

The above-mentioned, user driven expert review with annotation is merely one proposed training data provisioning scheme as may be supported by the training data provider system TDPS. Other set-ups or policies are likewise envisaged herein so long as the described pairing between *c', s*' and *p'* can be obtained.

Once a sufficient pool {(*c', s'), p'*)*ⁱ*} of such triple of data training data items is built up (with index i indicating an individual such training data instance), which can be in tens or hundreds per entity, this data can then be fed into, for example, a supervised training algorithm and processed as is otherwise understood. For example, the training input data *x=(c', s')* may be fed into a convolutional neural network model, or any other model, such as decision tree, regression mode, etc., to obtain a prediction output, *M^{θ}(x)* which can then be compared with the associated target *y=p'.* The difference *Dist[(M^{θ}(x),y)]* between the two, training output *M^{θ} (x)* vs target *y*, may be used to construct a cost function that is to be minimized in an optimization procedure. As an alternative, and indeed preferably, in order to reduce data dimensions, setup (1) may re-parameterized, *p=p(q)* by using the associated objective parameters. The optimization (1) is then run over *q*-space, rather than *p*-space. The remapping from *q* to *p* may then be done in a rules-based fashion, via LUTs, or via a separately trained ML model (not shown) may be learned for this, using the same ML principles are desorbed herein, only that the training data now includes pairs of (*q', p'*). Thus, model M may provide the imaging settings explicitly or implicitly, via the *q* values, as needed.

The optimization procedure may proceed iteratively according to a numerical algorithm, in order to adapt current model parameters *θ* of the model *M^{θ}* (either initial or pre-trained), so as to improve the cost function, that is, in generally to decrease the error *Δ*, or at least ensure error is not increasing. The model parameters θ may be adapted based on the gradient of the cost function, *grad F,* such as in any gradient descent scheme. This model parameter θ adaptation is done for some or all of the training data (such as in "batch(es)"), until a stopping condition is fulfilled in which case the model is considered trained.

As mentioned, the scale *S* or score scale may be individually drawn up for each entity, such as for each user, and the model may be trained separately for each user resulting in a bank of models for users k at a medical facility and such bank of models {*Mₖ*}may be held in a memory. In use (that is, in inference or test phase, after training), the individual user k, once logging-on with their credentials can have their respectively trained model accessed based on the credentials, and their personally trained model hen then be used for predicting imaging settings, as described above. If the entity is not a particular user, that is a medical person but say a medical facility collectively, a generally agreed score can be used or an average of individually compiled scores may be used to so form one single model for such a collective entity, as needed.

Training of the machine learning model M in the above or other setup may be a one-off, but, more usefully, may be (re-)done repeatedly, periodically or on demand, as new training data in terms of number and/or variation emerges. For example, at a clinical site during ML model deployment (inference), if the system is observed to operate sub-par it could indicate that the image scenarios currently dealt with are unfamiliar with the machine learning model. A re-training may be called for by user, such as be requested by user through the, or a, user interface. Thus, user may switch over the system IF into learning/training mode. The new data may be annotated by user on the spot, such as by awarding personal IQ score s'. These are then stored in association with the current context data, together with current imaging settings *p'* as target. In this manner, new training data is build up, and, once a sufficient stock of such training data is accrued, training may commence, using the current trained model as a pre-trained model on which training on the new data is then based, and so on in future training cycles.

Training data *d* is in general based on imagery, either synthetically generated by generated machine learning models, such as GAN (generative adversarial network)'s or others, or, may be based on historical imagery as may have been obtained in previous exams of the same or other patients at a medical site, such as a GP practice, a clinic, hospital or group of hospitals or a group of medical facilities or at any medical facility in general. However, in the present system, such imagery itself is not part of the training data as such (and is not part of the input during inference). Thus, in the present setup, the training imagery is more used for review, for score awarding, but the imagery itself is not provided as input to the model for processing, neither in training nor in testing/inference. The said imagery is merely used for visualization so that experts can annotate the training data, such as awarding scoring which then forms part of the training data input. The context data, also part of the training data input, and the corresponding imaging setting (which forms the target/ground truth) can be obtained from databases, metadata, etc. However, it is not excluded herein in some other embodiments that the imagery itself is also processed in an additional channel of the model, alongside context data and score, as additional (image-based) context data. However, for better responsiveness, processing of image data is not required herein. The processing scoring, context and imaging setting data suffices, it has been found. This "imageless" processing eases demand on memory and CPU requirements. Thus, whilst the model is trained herein for predicting imaging settings, image data itself need not be processed.

As to the model architecture M itself, this may be any regression type model that regresses input into output, namely the imaging setting. However, sometimes, instead of the model providing the exact imaging setting, it may be sufficient for classifying the input into any one of a number of different buckets or intervals. For example, instead of the model predicting the voltage setting of the tube SS in terms of the exact voltage, it may be sufficient to predict an interval (out of plural voltage intervals) into the which the voltage to use ought to fall

In embodiment, an architecture for model M may include for example an artificial neural network ("NN"), for example of the convolutional type ("CNN"). The training algorithm may be gradient descent based, such as backpropagation methods, or other.

Such NN type model M is made up of a set of computational nodes arranged in cascading layers, with nodes in one layer passing their output as input to nodes in a follow up layer. Much of the following description of an NN type model applies to training and inference/testing, which is why occasionally there will be reference to input *(c,s)* and output *p,* instead of the primed notation (*(c',s'), p')* for training data, as opposed to testing/inference data (*(c,s), p).*

The NN model M may be said to have a deep architecture because it has more than one hidden layer. In a feed-forward network, the "depth" is the number of hidden layers between input layer and output layer, whilst in recurrent networks the depth is the number of hidden layers, times the number of passes.

The layers of the network, and indeed the input *x=(c',s')* and output dataM(x), and the intermediate input and output between hidden layers (referred to herein as feature maps), can be represented as two or higher dimensional matrices ("tensors") for computational and memory allocation efficiency. The score *s',* s and the contextual data *c', c* may be presented, after suitable embedding, as a vector as mentioned earlier. This vector may be replicated to form a matrix or tensor, and this is then passed on in this matrix/tensor form as input into model. Hower, input as vectors (a 1-dim matrix) is also envisaged herein. Representation in terms of matrices/vectors may be for computational and memory allocation efficiency, as mentioned earlier in connection with SIMD.

Preferably, the hidden layers include a sequence of convolutional layers. The number of convolutional layers is at least one, but a plurality is preferred, such as 2-5, or any other number, for example in the 10s or in the 100s or higher still.

In training, but also in inference/testing, input data *x=(c', s')* is applied to input layer. The input data *x* then propagates through a sequence of hidden layers (they may be only one hidden layer, but in deep learning there may be more than one), to then emerge at output layer OL as an estimate output M(x), which represents imaging setting, either for training or as final output in inference.

In embodiments, downstream of the sequence of convolutional layers, and upstream the output layer, there may be one or more fully connected layers, in particular if a regression result is sought. The output layer ensures that the output has the correct size and/or dimension.

Preferably, some or all of the hidden layers are convolutional layers, that is, include one or more convolutional filters which process an input feature map from an earlier layer into intermediate output, sometimes referred to as logits. An optional bias term may be applied by addition for example. An activation layer processes in a non-linear manner the logits into a next generation feature map which is then output and passed as input to the next layer, and so forth. The activation layer may be implemented as a rectified linear unit (RELU), or as a *soft-max*-function, a sigmoid-function, tanh-function or any other suitable non-linear function. Optionally, there may be other functional layers such as pooling layers or drop-out layers (not shown) to foster more robust learning. The pooling layers reduce dimension of output whilst drop-out layer sever connections between nodes from different layers.

Thus, model M, for example in the said NN architecture, may be configured for multi-channel processing. For example, the model M may be configured to process a matrix including the score and the context data. Convolutional operators may operate separately on each channel, or preferably, there is cross-channel convolution to combine, in the course of (forward)propagation through the network M, information from the data types *s',c'* or *s,c* in inference. Having thus described an NN type ML model, such is not at the exclusion of other ML models, such as clustering type algorithms, including nearest neighbors, or others. Yet other ML techniques, such as statistical regression techniques, decision trees, random forests, support vector machines, etc., may be used instead or in addition, at least in part(s) for the ML pipeline M.

Referring now in more detail to the training data, it will be understood that the training data d includes a specific indication of such a personalized image score *s',* as mentioned earlier. The training data *d=(c', s', p')* is in general different from deployment data "seen" by the trained model in use after training, such as in testing or deployment. The training procedure, that is, the finding of the trainable parameters of the model so that model "fits" the training data can be understood as a sampling of a high dimensional energy surface defined by the cost function. Model parameters θ (such as weights of an NN type model) are adapted using the score s as an additional input parameter channel, that regularizes the training. The score allows the model to account for the clustered structure in the target space Π as indicated above in Fig. 3. In other words, "enriching" training data *(c',p'*) into an enlarged set *(c',s',p')* by adopting the additional score parameter S', facilitates robust, efficient, training. Such "s-enriched" data facilitates training algorithm to navigate the clustered landscape in parameter space Π, as encapsulated by the training data *(c', s', p').* In deployment, after training, the providing as input of score s in addition to the context data helps the model to better find the personalized imaging setting *p.*

The personalized IQ score s may be defined to vary over a scale *S.* The scale for the personalized IQ preference score for subjective image quality may differ from user to user. Thus, the described models may be trained separately for each such entity, each with their own scale for parameterizing imaging quality into personalized image quality. Thus, there may be a bank of such models for different users, with some or each model trained and regularized by a personalized scale of image scores for a particular user.

Reference is now made to Fig. 6, which shows a flow chart of a method of controlling or facilitating an imaging operation as envisaged herein in embodiments, based on a trained machine learning model as described above in the previous Figures and in any one of the related embodiments. However, it should be noted that the below described steps are not necessarily tied to the architectures described above, and the method may be understood as a teaching in its own right.

At step S610 an imaging request R is received from the user in text, audio (speech), or any other format provided through a suitable user interface. However, such imaging request need not necessarily be supplied explicitly. The user operating the imaging apparatus, such as triggering an acquisition (e.g., via pedal operation) is sufficient as such. Such operating may trigger the suppling of the request data *R.*

The request R may include input data *D.* The input data *D* may include score data *s* and context data c being supplied by the system or user. Context data *c* may include in particular imaging geometry settings, such as when user adjusts for a projection direction for instance. Thus, whilst in general, imaging geometry data may be part of the imaging settings to be computed herein, in some embodiments a part of the imaging settings may be in fact part of the context data *c.*

It is preferred that the method may proceed in an automated fashion. Specifically, the method may include interfacing with suitable one or more nodes (such as the imager **IA** itself, its operating console, data storage, data communication infrastructure COM, etc.), to retrieve the needed data *(c,*s) automatically, without explicit user interaction needed at all. This alleviates user, in particular in potentially stressful medical use cases.

At step, S620, the input data *D* as per the request *R,* if not already numerical, may be transformed into suitable numerical form for processing, such as by an embedding algorithm that maps input data into elements of a vector space, for example. It is such embedded, or transformed, data, that is then provided as input data *D.*

At step S630, the input data *D=(c,s)* is received, implicitly (preferred) or explicitly. Such data D includes a component for the image context c. The context data *c* may relate to a certain phase of a procedure which is to be image-guided. Thus, context data c may be indicative of a given procedure, or phase thereof, imaging purpose, etc. The input data *D* further includes a data component that is indicative of the personalized image quality score *s,* that may represent a personal preference of image quality.

The context data *c* may include data on the imaging, such as its purpose, and patient data on the patient to be imaged. The subject (such as the said patient data) may include patient's (bio-) details. This data may be provided explicitly or implicitly. For example, patient ID information (name, patient number, etc.) may be sufficient, as related patient bio-data may be pulled from a patient database, registry, HIS (hospital information system), etc., via suitable database interfacing, enabling information retrieval operations.

Context data *c* and score *s* is passed as input to a trained machine learning model M for processing by such model M at step S640. Specifically, data c and s are co-processed by the trained machine learning model M to obtain a personalized imaging setting *p* at step S650, as "modulated" by score *s.*

It can be seen herein that the provision of a separate personalized IQ score s in correspondence with Fig. 3 above constitutes an instance data-based/driven regularization of the machine learning. Score s, in general a scalar value, and variable along a scale S, provides an additional data space dimension for learning, over and above the dimension of the context data *c.*

A scalar dimension of *1* for score s suffices, but could be of higher dimension, whilst the context data c is in general of higher dimension, with *dim* >*1*, in practice much higher, possibly in the tens or 100s, and similar for the dimension of the imaging setting p, which may also run into the 10s or 100s.

At step S650 the model output *p* is provided for consumption. In addition to the output imaging setting *p,* the initially input intended score *s* can also be provided as output, such as being displayed alongside the imaging setting *p.*

For example, at step S660 the so produced and provided imaging setting/instruction(s) *p* may be used in an imaging procedure, such as applied to the imager to reset some of the imager IA's components (such as one or more of the components described above at Fig. 2).

At a next step S670, the imaging procedure is then carried out, based on the settings/instruction(s) *p,* to so produce imagery by imager IA at user personalized IQ as per the score s received at step S610.

As mentioned earlier, the model may comprise a bank of models, that is, one model trained per user based on their respective scoring scale *S∈s*.

The above can be repeated for a next imaging run, and so on, with the user providing new input data D', with at least partly new context data *c*", and/or new IQ score *s,* as needed. The new/updated context data *c*" may include an updated imaging purpose etc., for example indicative of a different phase of the current procedure, etc., whilst the patient related data (e.g., the said bio-characteristics) may be maintained. The method may be used in times series imaging, live action feeds *mₜ,* etc., such as in IGPs or other.

In some embodiments, in order to reduce. e.g. minimize, the dose level to a personal level, the system could lower the image quality and dose level by a certain amount after a certain number of cases. As soon as user provides negative feedback on image quality (either via direct input or via other staff members), the model may be provided with this feedback, thus causing to increasing the dose level somewhat, to improve image quality at an optimized dose level.

Reference is now made to Fig. 7 which shows a flow chart of a method for training the machine learning model as envisaged herein in embodiments and for use in imaging facilitator IF.

At step S705, training data provided. Historical imaging logs, (metadata of) imagery, header data, etc., patient pose imagery, etc., may be accessed for this. For pre-training, a text corpus of a suitable general domain knowledge is provided, such a medical imaging.

The providing S705 of training data may include: displaying existing (e.g., medical) imagery to user or expert, receiving from user or expert an annotation that indicates a score for personal IQ in relation to the displayed image, and obtaining imaging settings that were in place when the display image was acquired, as well as contextual data in relation to the imaging for the displayed image, and storing the triple i)-iii) of data items ( i) context data c' ii) score *s*', and iii) imaging setting *p'* ) to obtain a training data item. The above may be repeated for each or some such displayed image to so built up a corpus of training data triples. The imaging stetting in each triple may be used as target *y* for the training data input x comprising the other two items i),ii) from the given triple, and so on for some or each such triples.

At step S710. Training data is received, either as provided at step S705, or whichever way.

Based on the training data, an ML model is trained. The said training may be based on adjusting parameters of the model, based on operation of the model on the training data. The adjusting of the model parameters may be based on an objective function F, such as a cost function. The parameters are adjusted to improve the cost function. In more detail, the training step may include one or more of the following:-
At step S720, in any given training cycle, the training input *x*ₖ of an instance *k* is applied to an initialized or earlier trained machine learning model M to produce training output *M*(*xₖ*)*.*

An error of the training output *M*(*xₖ*) from the associated target *yₖ* is quantified by cost function *F.*

One or more parameters of the model are adapted at step S730 and the errors evaluated at S740 in one or more iterations in an inner loop to improve the cost function. For instance, the model parameters are adapted to decrease errors as measured by the cost function. Herein, index k may refer to individual instants/samples, such as pairs, of a given batch (subset) of training data, or the index may refer to the batch as a whole, or indeed to the whole training data, as needed.

Once sufficient convergence is established, e.g. by observing the values returned by cost function F (which a real valued function), the training method then returns in an outer loop to step S720, where the next batch of training data pairs is fed in. Forward and/or Backpropagation techniques may be used, or other gradient-based techniques may be used in the inner loop.

As mentioned above, in particular the contextual data c' or imaging setting data *p*' may not necessarily be natively numeric but need to be processed by a data preparation module (not shown), which converts non-numeric data into numeric data such as co-ordinates of a, possibly high dimensional, vector in a respective space, such as in imaging setting spaces or patient characterization spaces, etc. The order of the co-ordinates in each such vector, such as may be obtained by using existing embedding algorithms, is immaterial, but should preferably be retained for training and for ML deployment to ensure best results. Specifically, the embedder is operable not only in training, but also during deployment/inference or testing, before feeding the input data into the model for processing during deployment/testing. This data preparation can be done up-front or can be done on demand as needed, on-the-fly.

### Use case

The below pertains to an illustration of the above-described principles with reference to a non-limiting explain use case. Such use case may pertain to a PCI-procedure (percutaneous coronary intervention). Such may comprise plural phases. Some examples of such phases may include any one or more of:
- navigation of the catheter towards the coronary arteries;
- imaging the coronary arteries with iodinated contrast medium;
- navigating the catheter to the occlusion;
- inflating a balloon and or placing a stent.

The objective image quality needed for those phases may vary greatly, as interventionalists need to identify object details or anatomies of different spatial resolutions or contrasts within the images. Apart from that, the objective image quality itself will depend upon the projection of the X-ray beam through the patient and anatomical and physiological aspects, like artery size, blood flow and patient size.

The subjective image quality needs to be "optimal", meaning that imaging is acceptable for an individual user at the lowest possible X-ray dose. Note that users have a different (and very individual) perception of what is "acceptable image quality", which also depends on their experience. Only the user (attending physician) can determine what level and "flavor" of image quality is needed to optimize the clinical task at hand. Generally speaking, the subjective image quality, or IQ score s may the held constant for a given user. The score may correspond to "acceptable image quality" for the given user.

The system FS described herein is capable of adjusting the imaging setting of the cath lab XX-ray imager IA in such a way that the image quality for a particular user is such that details necessary for the PCI can be distinguished in the resulting images, thus being able to perform the upcoming clinical task or phase thereof. In PCI, such task/phase may include navigating the catheter, assessing the occlusion, or observing the stent expand against the vessel wall. The system FS may operate to automatically adapt the settings for variations in patient size, blood flow, etc. The system FS may attempt to optimize the settings with respect to patient dose. The system FS may make such adjustments according to the IQ preference s of the current user.

The system FS can be trained with input from one or plural users, providing respective training input in form of the score s' for a set of clinical procedure(s)/context(s) c'. After training, in order to personalize the system FS, (additional) input may be provided by a single user in form of their score s, to so adjust system or given user's preferences. After training, the user may interact with the trained system FS, for example to request imaging with increased image sharpness, increased contrast of in-image representation of medical device(s), or preferred personal "flavors". The trained system can provide information to the user about the proposed imaging setting p, and optionally on the image quality, in relation to the upcoming run/frame.

Components of the imaging facilitator IF may be implemented as one or more software modules, run on one or more general-purpose processing units PU such as a workstation associated with the imager IA, or on a server computer associated with a group of imagers.

Alternatively, some or all components of the imaging facilitator IF may be arranged in hardware such as a suitably programmed microcontroller or microprocessor, such an FPGA (field-programmable-gate-array) or as a hardwired IC chip, an application specific integrated circuitry (ASIC), integrated into the imaging system IA. In a further embodiment still, the imaging facilitator IF may be implemented in both, partly in software and partly in hardware.

The different components of the imaging facilitator IF may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A system (FS) of facilitating operation of an imaging apparatus (IA), comprising:-
input interface (IN) for receiving input data (D) comprising i) imaging context data (c) relating to an intended imaging procedure or to a phase of such a procedure and/or to a subject (PAT) to be imaged, and ii) image quality, IQ, score data (s) indicative of an intended IQ to be achieved, and
a trained machine learning model (M) configured to process the input data (D) to compute output data (*p*) related to an imaging setting for the imaging apparatus (IA).

2. A system of claim 1, wherein said score (s) represents an imaging preference from a scale of such preferences of a user, department, or organization.

3. A system of claim 1 or 2, wherein the input data is so receivable in an ongoing imaging procedure, and wherein the imaging setting is one for acquisition of a next frame in the ongoing imaging procedure.

4. A system of claim 3, wherein the imaging context as per the input data is subject to change in the ongoing imaging procedure, wherein the trained machine learning model (M) to compute updated such imaging setting per such change.

5. A system of any one of the preceding claims, wherein the said input data is suppliable by user via a user interface (UI).

6. A system of any one of the preceding claims, wherein the system includes an output interface (OUT) for supplying the said imaging setting.

7. A system of any one of the preceding claims, wherein output interface (OUT) is capable of interfacing with control circuitry (CC) of the imaging apparatus to thereby cause the imaging setting being applied to the imaging apparatus, thus enabling the imaging apparatus to acquire one or more frames at the said imaging setting.

8. A system of any one of the preceding claims, wherein, on the system so supplying the imaging setting and the imaging apparatus acquiring a current frame at the supplied imaging setting, the input interface (IN) to receive further input data including a user feedback on the current frame, and the trained machine learning model (M) further capable to compute, based on the feedback, a new imaging setting for a subsequent frame.

9. A system of any one of the preceding claims, wherein i) the imaging apparatus is of the interventional type, and/or ii) wherein the imaging apparatus is X-ray based, and/or iii) wherein the procedure is an image-guided procedure.

10. An imaging arrangement (MIA), comprising at least parts of the system (FS) as per any one of the preceding claims, and further comprising any one or more of: the imaging apparatus (IA), the user interface, data storage on which is stored at least some parameters of the trained model.

11. A computer-implemented method of facilitating operation of an imaging apparatus (IA), comprising: -
receiving (S630) input data (D) comprising i) imaging context data (c) relating to an intended imaging procedure or to a phase of such a procedure and/or to a subject (PAT) to be imaged, and ii) image quality, IQ, score data (s) indicative of an intended IQ to be achieved, and
by trained machine learning model (M), processing (S640) the input data (D) to compute output data (*p*) related to an imaging setting for the imaging apparatus (IA).

12. A method of training, based on training data, a machine learning model to obtain the model as per any one of the preceding claims.

13. A method of providing training data on which the model in claim 12 is trainable.

14. A computer program element, which, when being executed by at least one computing system, is adapted to cause the computing system to perform the method as per claim 11, 12 or 13.

15. At least one computer readable medium having stored thereon the program element of claim 14.
